# EUROPÄISCHE PATENTSCHRIFT

(11) **EP 2 572 754 B1**
(45) Veröffentlichungstag und Bekanntmachung des Hinweises auf die Patenterteilung: **23.03.2016**
(21) Anmeldenummer: 12179327.7
(22) Anmeldetag: 06.08.2012
(51) Int. Cl.: A61N 5/00, A61N 5/10

(54) **Verfahren und Vorrichtung zur Bestimmung der Bestrahlungsdauer bei einer Partikelbestrahlungsplanung**
Method and device for determining the radiation duration of a particle radiation plan
Procédé et dispositif de détermination de la durée de rayonnement lors d'une planification d'irradiation de particules

(30) Priorität: 22.09.2011 DE 102011083196
(43) Veröffentlichungstag der Anmeldung: 27.03.2013
(73) Patentinhaber: Siemens Aktiengesellschaft, 80333 München (DE)
(72) Erfinder: Gemmel, Alexander, 91054 Erlangen (DE); Elsässer, Thilo, 91054 Buckenhof (DE); Krebs, Philipp, North Vancouver, BC, V7L 2H2 (CA)

(56) Entgegenhaltungen:
- WO-A1-2009/052845
- JP-A- 2004 337 371
- US-A1- 2009 134 345
- HARALD PAGANETTI ET AL: "Clinical implementation of full Monte Carlo dose calculation in proton beam therapy", PHYSICS IN MEDICINE AND BIOLOGY, INSTITUTE OF PHYSICS PUBLISHING, BRISTOL GB, Bd. 53, Nr. 17, 7. September 2008 (2008-09-07), Seiten 4825-4853, XP020141398, ISSN: 0031-9155, DOI: 10.1088/0031-9155/53/17/023

## Beschreibung

Die Erfindung betrifft ein Verfahren sowie eine Vorrichtung zur Bestimmung der Bestrahlungsdauer bei einer Bestrahlungsplanung.

In der Strahlentherapie wird unter anderem erkranktes Gewebe mit Röntgenstrahlen, mit Elektronenstrahlen oder mit Partikelstrahlen bestrahlt. Insbesondere die Partikeltherapie entwickelt sich in den letzten Jahren zu einem etablierten Verfahren zur Behandlung von Gewebe, insbesondere von Tumorerkrankungen. Bestrahlungsverfahren, wie sie im Rahmen der Partikeltherapie eingesetzt werden, können jedoch auch in nicht-therapeutischen Bereichen eingesetzt werden, wie beispielsweise bei der Bestrahlung von Phantomen oder nichtlebenden Körpern im Rahmen von Forschungsarbeiten, bei der Bestrahlung von Materialien, etc.

Bei der Partikeltherapie werden Partikel erzeugt, vornehmlich Ionen wie Protonen, Kohlenstoffionen, oder andere Ionensorten. Diese Partikel werden auf hohe Energien in einem Beschleuniger beschleunigt, zu einem Partikelstrahl geformt und anschließend auf das zu bestrahlende Gewebe gerichtet. Die Partikel dringen in das zu bestrahlende Gewebe ein und geben dort in einem umschriebenen Bereich ihre Energie ab. Die Eindringtiefe des Partikelstrahls in das zu bestrahlende Gewebe hängt vornehmlich von der Energie des Partikelstrahls ab. Je höher die Energie des Partikelstrahls ist, desto tiefer dringen die Partikel in das zu bestrahlende Gewebe ein.

Während der Bestrahlungsplanung ist die zu erwartende Abstrahldauer des Plans und seiner einzelnen Felder/Beams unbekannt. Durch Einstellung der Optimierungsparameter wird nach dosimetrischen Gesichtspunkten eine Dosisverteilung optimiert und ein Fraktionsschema festgelegt, so dass die Bestrahlungszeit, die sich auf den Patientenkomfort und -durchsatz sowie auf die Strahlenbelastung der Patienten auswirkt, nicht feststeht. Hinzu kommt, dass sich Variationen in der Beschleunigerperformance unterschiedlich stark auf die Bestrahlungsdauer auswirken können.

Die Faktoren, die die Bestrahlungsdauer eines Feldes/Beams beeinflussen, sind qualitativ bekannt. Hierzu zählen neben der Performance des Strahlapplikationssystems bzw. des Beschleunigers, die sich nicht durch die Bestrahlungsplanung beeinflussen lässt, die minimale Teilchenzahl, der Schichtabstand und die Größe des zu behandelnden Zielbereichs z.B. eine Tumorgröße. Die Tumorgröße selbst ist nicht anpassbar; durch Anpassung des Feldwinkels/Einstrahlwinkels lassen sich jedoch die Ausdehnung des Tumors in der Tiefe relativ zur Feldrichtung verändern und damit die notwendige Anzahl der Schichten zur Abdeckung des Tumors.

Die quantitative Auswirkung der Faktoren ist jedoch patientenabhängig. Beispielsweise kann eine Erhöhung der minimalen Teilchenzahl in einem Fall zu einer Zeitersparnis von 15 Prozent führen, während sie in einem anderen Fall nur drei Prozent beträgt und damit im Bereich der erwartbaren Schwankungen der Beschleunigerperformance liegt. Da eine Erhöhung der minimalen Teilchen-/Partikelzahl üblicherweise mit einer Verschlechterung seiner klinischen Kenngrößen (Tumorabdeckung, Max. Strahlendosis) einhergeht, ist derzeit eine Abwägung des zeitlichen Nutzens und der dosimetrischen Auswirkung nicht möglich.

US 2009/0134345 A1 betrifft ein Tool zur Bestrahlungsplanung bei der Schwerionen-Therapie. Die grundliegende Idee dort ist es, nachdem das Konzept des verwischten bzw. verteilten Bragg-Peaks (SOBP) bekannt ist, beliebige Dosisverteilungen kreieren zu können, indem mehrere aufeinanderfolgende Anwendungen unterschiedlicher SOBP-Strahlprofile eingesetzt werden. Es werden verschiedene Modelle der SOBP-Dosisprofile mit unterschiedlichen Energien und/oder Behandlungszeiten mathematisch kombiniert, so dass ein Behandlungsprofil entsteht, das nicht mit nur einem einzigen SOBP-Dosisprofil erhaltbar wäre. Entsprechend werden die verwendeten SOBP-Modelle, die Energien und die Behandlungszeiten ausgegeben und bilden also den Bestrahlungsplan.

Es ist die Aufgabe der Erfindung, eine Vorrichtung sowie ein Verfahren zur Planung einer Bestrahlungsbehandlung anzugeben, wobei eine möglichst genaue Bestrahlungsdauer bestimmt werden soll.

Die Aufgabe wird mit einem Verfahren sowie einer Vorrichtung und einem Computerprogrammprodukt gemäß den unabhängigen Patentansprüchen gelöst. Vorteilhafte Ausgestaltungen des Verfahrens sowie der Vorrichtung und des Computerprogrammprodukts sind Gegenstand der abhängigen Patentansprüche oder lassen sich aus der nachfolgenden Beschreibung sowie den Ausführungsbeispielen entnehmen.

Das erfindungsgemäße Verfahren zur möglichst genauen Abschätzung der Abstrahlzeit unter Berücksichtigung verschiedener Beschleunigereffizienzen zum Zeitpunkt der Bestrahlungsdauer zu verwenden. Es verwendet Plandaten bzw. Behandlungsgrößen wie Schichtanzahl und Teilchenzahl bzw. Partikelenergie sowie Partikelbeschleunigerkenngrößen wie Beschleunigungszeit, Spilllänge und Intensitätsstufen der Teilchenabstrahlung und Regeln der Prozessdatengenerierung, um eine exakte Abschätzung der Bestrahlungsdauer mit einer Genauigkeit von 1%+/-4% zu geben. Zusätzlich lassen sich Abschätzungen für den besten Fall (best case) bzw. schlechtesten Fall (worst case) machen, um den tagesabhängigen Schwankungen in der Beschleunigereffizienz Rechnung zu tragen.

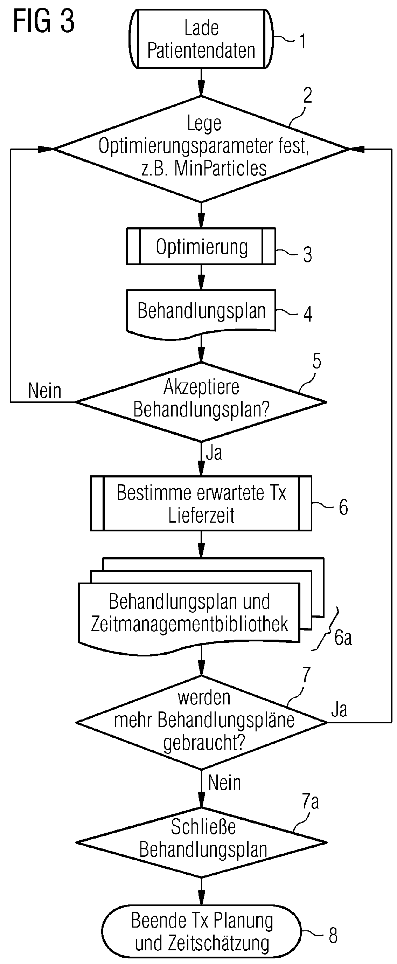

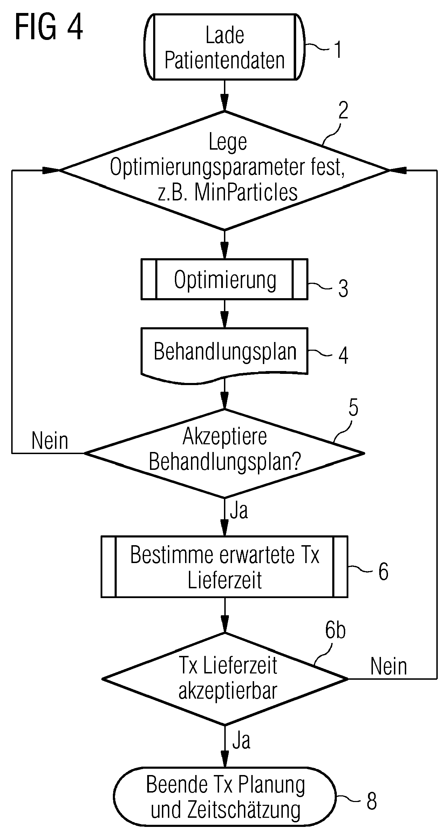

Das erfindungsgemäße Verfahren - wie in den Figuren 3 und 4 schematisch dargestellt - kann während der Bestrahlungsplanung und nach der Planoptimierung eingesetzt werden. Es könnte als Modul der Bestrahlungsplanung oder als eigenes Programm laufen. Beispielsweise - wie nachstehend zu den Figur 3 und 4 erläutert - sind unter anderem folgende Arbeitsabläufe (Workflow-Szenarien) denkbar:
1. Bildung einer Mehrzahl von Plänen, für die die Bestrahlungszeit berechnet wird und deren Auswahl durch einen Vergleichsmodus erfolgt,
2. Bildung eines Plans und Entscheidung, ob die erwartbare Bestrahlungsdauer (Mittelwert, Worst/Best case) zufriedenstellend ist.

Erfindungsgemäß wird eine gezielte Auswahl eines Plans unter Berücksichtigung der erwartbaren Bestrahlungsdauer bei der Bestrahlungsplanung zur Erhöhung des Patientenkomfort und - durchsatz erreicht. Zudem wird bei der Bestimmung der Bestrahlungsdauer die Genauigkeit der Abschätzung verbessert.

Ein weiterer Aspekt der Erfindung ist eine Vorrichtung zur Bestimmung der Bestrahlungsdauer bei einer Partikelbestrahlungsplanung aufweisend Mittel bzw. Module zur Durchführung des oben genannten Verfahrens, die jeweils hardwaremäßig und/oder softwaremäßig bzw. als Computerprogrammprodukt ausgeprägt sein können.

Die Vorrichtung kann in einer Rechnereinheit implementiert sein.

Die Vorrichtung und das Computerprogrammprodukt können wie das Verfahren entsprechend weitergebildet werden.

Weitere Vorteile, Einzelheiten und Weiterbildungen der Erfindung ergeben sich aus der nachfolgenden Beschreibung von Ausführungsbeispielen in Verbindung mit den Zeichnungen.

Es zeigen:
Fig. 1 einen schematischen Aufbau einer Partikeltherapieanlage,
Fig. 2 eine Anordnung außerhalb bzw. innerhalb eines Bestrahlungsraums und
Figur 3 schematisch ein Ablaufdiagramm zum erfindungsgemäßen Vorgehen, wobei eine Mehrzahl von Bestrahlungsplanen erstellt wird und
Figur 4 schematisch ein Ablaufdiagramm zum erfindungsgemäßen Vorgehen, wobei ein Bestrahlungsplan erstellt wird.

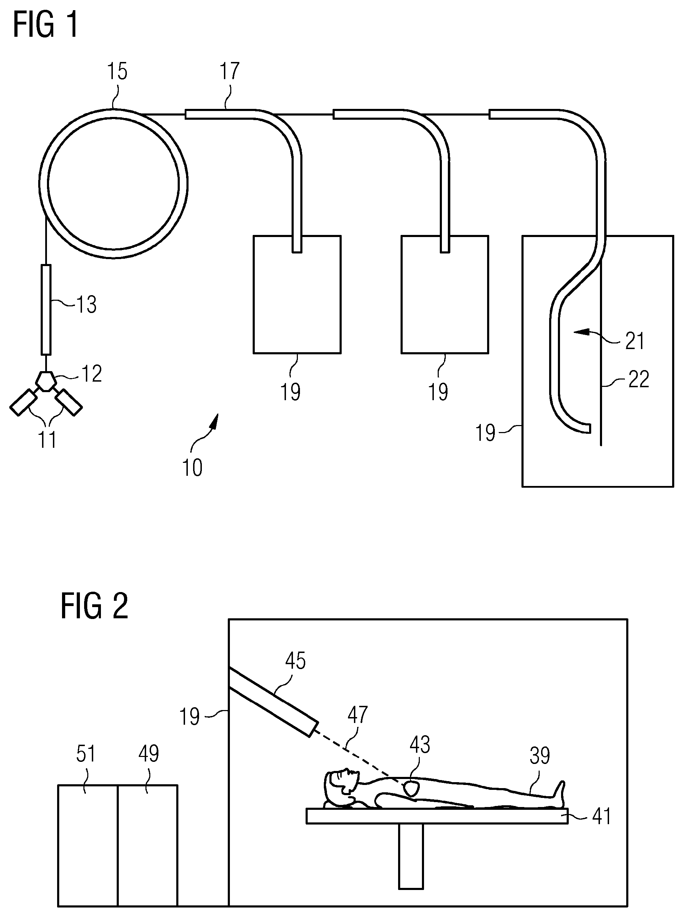

Die Figuren 1 und 2 zeigen schematisch eine Partikeltherapieanlage und eine Anordnung außerhalb bzw. innerhalb eines Bestrahlungsraums, wie sie beide beispielsweise aus DE 10 2008 019 128 A1 bereits bekannt sind.

Fig. 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den (Partikel-)Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Aufbau einer Partikeltherapieanlage 10 ist typisch für viele Partikeltherapieanlagen, kann aber auch hiervon abweichen. Die nachfolgend beschriebenen Ausführungsbeispiele sind sowohl in Zusammenhang mit der anhand von Figur 1 dargestellten Partikeltherapieanlage als auch mit anderen Partikeltherapieanlagen einsetzbar.

Figur 2 zeigt eine mögliche Anordnung außerhalb bzw. innerhalb eines Bestrahlungsraums.

Eine in der Figur 2 nicht dargestellte Bildgebungseinheit umfasst in der Regel einen Röntgendetektor und einen Röntgenstrahler, die an einem Tragarm, z.B. einem C-Bogen, einander gegenüber liegend angeordnet sind. Der Tragarm selbst ist mithilfe eines Roboterarms, beispielsweise mithilfe eines sechsachsigen Knickarm-Roboters, im Raum flexibel positionierbar. Mithilfe des Röntgendetektors und des Röntgenstrahlers können Röntgenaufnahmen, zum Beispiel Durchleuchtungsaufnahmen, von einem Patienten 39 aufgenommen werden, der zur Bestrahlung auf einer Patientenliege 41 positioniert ist. Insbesondere den zu bestrahlenden Zielbereich bzw. das zu bestrahlende Zielvolumen 43, beispielsweise ein zu bestrahlendes, von einem Tumor befallenes Organ, kann in den Durchleuchtungsaufnahmen abgebildet sein.

Zur Bestrahlung tritt aus einem Strahlaustritt 45 ein Partikelstrahl 47 aus und ist auf den Patienten 39 gerichtet. Hier gezeigt ist ein im Raum räumlich fest installierter Strahlaustritt 45. Alternativ ist es auch möglich, den Strahlaustritt 45 an einer drehbaren Gantry zu befestigen, so dass der Strahlaustritt 45 um den Patienten 39 gedreht werden kann. Während der Applikation des Partikelstrahls 47 bleibt der Strahlaustritt 45 jedoch im Allgemeinen ortsfest.

Die Steuerungseinheit 51 und/oder die Rechnereinheit 49 zur Bildrekonstruktion können in einer einzigen Rechnereinheit implementiert sein oder auch auf verschiedene Untereinheiten aufgesplittet, als eigenständige Einheiten implementiert werden oder in einer Steuerungseinheit für die gesamte Partikeltherapieanlage.

In der Rechnereinheit 49 implementiert bzw. an die Rechnereinheit 49 direkt bzw. abgesetzt (remote) gekoppelt kann eine weitere in der Figur 2 nicht dargestellte Behandlungsplanungseinheit zur Planung der Bestrahlungsdauer verwendet werden, die hardware- bzw. softwaremäßig ausgebildet sein kann.

Im Folgenden werden mögliche erfindungsgemäße Arbeitsabläufe erläutert, deren Schritte in den Figuren 3 und 4 mit den Bezugszeichen 1, 2, 3, 4, 5, 6, 6a, 6b, 7, 7a, 8 gekennzeichnet sind:
Gemäß Figur 3 werden folgende Schritte ausgeführt:
   1 Lade Patientendaten
   2 Setze die Optimierungsparameter wie Behandlungsplangrößen fest
   3 Führe eine Optimierung der Optimierungsparameter aus
   4 Erstelle einen Behandlungsplan
   5 Prüfe den Behandlungsplan
      Wenn Behandlungsplan nicht akzeptabel ist, dann wiederhole Schritte 2 bis 5
   6 Wenn Behandlungsplan akzeptabel ist, dann werte die erwartete Bestrahlungsdauer aus
   6a Lege den Behandlungsplan in einem Datenspeicher ab, der mehrere Behandlungspläne umfassen kann
   7 Wenn noch weitere Behandlungspläne notwendig sind, dann wiederhole Schritt 2 bis 6a und 7
   7a Wenn keine weiteren Behandlungspläne notwendig sind, dann wird ein Behandlungsplan aus dem Datenspeicher vorgeschlagen und/oder ausgewählt.
   8 Beende die Behandlungsplanung und Auswertung der Bestrahlungssdauer

Der Ablauf in Figur 4 unterscheidet sich vom Ablauf in Figur 3 durch folgende Schritte:
Schritte 6a, 7 und 7a aus Figur 3 werden ersetzt durch Schritt
   6b Wenn die in Schritt 6 ausgewertete Bestrahlungszeit akzeptabel ist, dann fahre mit Schritt 8 fort. Wenn die ausgewertete Bestrahlungszeit nicht akzeptabel ist, dann wiederhole Schritte 2 bis 6 und 6b.

"Tx" bedeutet in den Figuren 3 und 4 "Treatment" (Behandlung).

Erfindungsgemäß kann der Wert der oben ausgewerteten, erwarteten Behandlungsdauer zur Einstellungsoptimierung eines Partikelbeschleunigers 15 verwendet werden, wie es in der Deutschen Patentanmeldung Nr. 102011083195.9 "Verfahren und Vorrichtung zur Optimierung eines Partikelbeschleunigers" mit gleichem Zeitrang der vorliegenden Anmeldung vorgeschlagen worden ist.

## Patentansprüche

1. Verfahren zur Bestimmung der Bestrahlungsdauer bei einer Bestrahlungsplanung, aufweisend folgende Schritte:
a) Optimierung von Behandlungsplangrößen, woraus min-destens ein Behandlungsplan mit unbekannter erwarteter Bestrahlungsdauer erstellt wird (2, 3, 4, 5), und
b) Ermittlung der erwarteten Bestrahlungsdauer aus dem Behandlungsplan (6) und mit Hilfe von Partikelbeschleuniger-Kenngrößen, **dadurch gekennzeichnet, dass** die Partikelbeschleunigerkenngrößen zumindest die Beschleunigungszeit, Spilllänge und Intensitätsstufe der Teilchenabstrahlung umfassen.

2. Verfahren nach dem vorhergehenden Anspruch, **dadurch gekennzeichnet, dass** eine Ausgabe des Wertes der erwarteten Behandlungsdauer aus einem zuvor ausgewerteten Behandlungsplan erfolgt.

3. Verfahren nach dem vorhergehenden Anspruch, **dadurch gekennzeichnet, dass** der ausgewertete Behandlungsplan vorgeschlagen und/oder ausgewählt wird (7a).

4. Verfahren nach einem der vorhergehenden Ansprüche, **dadurch gekennzeichnet, dass** die Behandlungsplangrößen zumindest die Partikelzahl und Partikelenergie umfassen.

5. Vorrichtung (49) zur Bestimmung der Bestrahlungsdauer bei einer Bestrahlungsplanung, aufweisend:
a) Mittel zur Optimierung von Behandlungsplangrößen, woraus mindestens ein Behandlungsplan mit unbekannter erwarteter Bestrahlungsdauer erstellt wird,
b) Mittel zur Ermittlung der erwarteten Bestrahlungsdauer aus einem vorgeschlagenen und/oder ausgewählten Behandlungsplan und mit Hilfe von Partikelbeschleuniger-Kenngrößen, und
c) Mittel zur Ausgabe des Wertes der ausgewerteten, erwarteten Behandlungsdauer, **dadurch gekennzeichnet, dass** die Partikelbeschleunigerkenngrößen zumindest die Beschleunigungszeit, Spilllänge und Intensitätsstufen der Teilchenabstrahlung umfassen.

6. Vorrichtung nach dem vorhergehenden Anspruch **dadurch gekennzeichnet, dass** sie Mittel zur Ausgabe des Wertes der erwarteten Behandlungsdauer aus einem zuvor ausgewerteten Behandlungsplan aufweist.

7. Vorrichtung nach dem vorhergehenden Anspruch, **dadurch gekennzeichnet, dass** der ausgewertete Behandlungsplan vorgeschlagen und/oder ausgewählt ist.

8. Vorrichtung nach einem der vorhergehenden Ansprüche 5-7, **dadurch gekennzeichnet, dass** die Behandlungsplangrößen zumindest die Partikelzahl und Partikelenergie umfassen.

9. Computerprogrammprodukt geeignet für eine Vorrichtung (49) nach einem der vorhergehenden Vorrichtungsansprüche, aufweisend einen auf einem Computer ladbaren und/oder aus-führbaren Programmkode, der folgende Schritte aufweist:
a) Bereitstellen mindestens eines optimierten Behandlungsplanes mit unbekannter erwarteter Bestrahlungsdauer, und
b) Ermittlung der erwarteten Bestrahlungsdauer aus dem mindestens einen Behandlungsplan und mit Hilfe von Partikelbeschleunigerkenngrößen, **dadurch gekennzeichnet, dass** die Partikelbeschleunigerkenngrößen zumindest die Beschleunigungszeit, Spilllänge und Intensitätsstufen der Teilchenabstrahlung umfassen.

10. Computerprogrammprodukt nach dem vorhergehenden Anspruch, **dadurch gekennzeichnet, dass** die Behandlungsplangrößen, woraus mindestens ein Behandlungsplan erstellt wird (2, 3, 4, 5), vor der Auswertung der erwarteten Behandlungsdauer optimiert werden.

11. Computerprogrammprodukt nach einem der vorhergehenden Ansprüche 9-10, **dadurch gekennzeichnet, dass** als weiterer Schritt eine Ausgabe des Wertes der erwarteten Behandlungsdauer aus einem zuvor ausgewerteten Behandlungsplan erfolgt.

12. Computerprogrammprodukt nach dem vorhergehenden Anspruch, **dadurch gekennzeichnet, dass** der ausgewertete Behandlungsplan vorgeschlagen und/oder ausgewählt wird (7a).

## Claims

1. Method for determining the radiation duration of a radiation plan, having the following steps:
a) optimising treatment plan variables, from which at least one treatment plan with an unknown, expected radiation duration is produced (2, 3, 4, 5), and
b) determining the expected radiation duration from the treatment plan (6) and with the aid of particle accelerator parameters, **characterised in that** the particle accelerator parameters comprise at least the acceleration time, spill length and intensity level of the particle emission.

2. Method according to the preceding claim, **characterised in that** the value of the expected treatment duration is output from a previously evaluated treatment plan.

3. Method according to the preceding claim, **characterised in that** the evaluated treatment plan is proposed and/or selected (7a).

4. Method according to one of the preceding claims, **characterised in that** the treatment variables comprise at least the particle number and particle energy.

5. Apparatus (49) for determining the radiation duration of a radiation plan, having:
a) means for optimising treatment plan variables, from which at least one treatment plan with an unknown, expected radiation duration is produced,
b) means for determining the expected radiation duration from a proposed and/or selected treatment plan and with the aid of particle accelerator parameters, and
c) means for outputting the value of the evaluated, expected treatment duration, **characterised in that** the particle accelerator parameters comprise at least the acceleration time, spill length and intensity levels of the particle emission.

6. Apparatus according to the preceding claim, **characterised in that** it has means for outputting the value of the expected treatment duration from a previously evaluated treatment plan.

7. Apparatus according to the preceding claim, **characterised in that** the evaluated treatment plan is proposed and/or selected.

8. Apparatus according to one of the preceding claims 5-7, **characterised in that** the treatment plan variables comprise at least the particle number and particle energy.

9. Computer program product suited to an apparatus (49) according to one of the preceding apparatus claims, having a program code which can be loaded and/or run on a computer, having the following steps:
a) providing at least one optimised treatment plan with an unknown, expected radiation duration, and
b) determining the expected radiation duration from the at least one treatment plan and with the aid of particle accelerator parameters, **characterised in that** the particle accelerator parameters comprise at least the acceleration time, spill length and intensity levels of the particle emission.

10. Computer program product according to the preceding claim, **characterised in that** the treatment plan variables, from which at least one treatment plan is produced (2, 3, 4, 5), are optimised prior to the evaluation of the expected treatment duration.

11. Computer program product according to one of the preceding claims 9 - 10, **characterised in that** as a further step, the value of the expected treatment duration is output from a previously evaluated treatment plan.

12. Computer program product according to the preceding claim, **characterised in that** the evaluated treatment plan is proposed and/or selected (7a).

## Revendications

1. Procédé de détermination de la durée d'irradiation lors d'une planification d'irradiation, comprenant les stades suivantes :
a) optimisation de grandeurs de plan de traitement à partir desquels on établit (2, 3, 4, 5) au moins un plan de traitement d'une durée d'irradiation escomptée inconnue et
b) détermination de la durée d'irradiation escomptée à partir du plan (6) de traitement et à l'aide de grandeurs caractéristiques d'accélérateur de particules, **caractérisé en ce que** les grandeurs caractéristiques d'accélérateur de particules comprennent au moins le temps d'accélération, la longueur de rétrodiffusion et le degré d'intensité de l'émission des particules.

2. Procédé suivant la revendication précédente, **caractérisé en ce qu'**on effectue une sortie de la valeur de la durée de traitement escomptée à partir d'un plan de traitement exploité auparavant.

3. Procédé suivant l'une des revendications précédentes, **caractérisé en ce que** l'on propose et/ou on choisit (7a) le plan de traitement exploité.

4. Procédé suivant l'une des revendications précédentes, **caractérisé en ce que** les grandeurs du plan de traitement comprennent au moins le nombre de particules et l'énergie des particules.

5. Dispositif (49) de détermination de la durée d'irradiation lors d'une planification d'irradiation comprenant :
a) un moyen d'optimisation de grandeurs de plan de traitement à partir desquelles on établit au moins un plan de traitement ayant une durée d'irradiation escomptée inconnue.
b) un moyen de détermination de la durée d'irradiation escomptée à partir du plan de traitement proposé et/ou sélectionné et à l'aide de grandeurs caractéristiques d'accélérateur de particules et
c) des moyens de sortie de la valeur de la durée de traitement escomptée exploitée, **caractérisé en ce que** les grandeurs caractéristiques d'accélérateur de particules comprennent au moins le temps d'accélération, la longueur de rétrodiffusion et des degrés d'intensité de l'émission des particules.

6. Dispositif suivant la revendication précédente, **caractérisé en ce qu'**il comprend des moyens de sortie de la valeur de la durée de traitement escomptée à partir d'un plan de traitement exploité auparavant.

7. Dispositif suivant la revendication précédente, **caractérisé en ce que** le plan de traitement exploité est proposé et/ou sélectionné.

8. Dispositif suivant l'une des revendications précédentes 5 à 7, **caractérisé en ce que** les grandeurs du plan de traitement comprennent au moins le nombre de particules et l'énergie des particules.

9. Produit de programme d'ordinateur approprié pour un dispositif (49) suivant l'une des revendications de dispositif précédentes, comprenant un code de programme pouvant être chargé et/ou exécuté sur un ordinateur, qui a les stades suivantes :
a) on se procure au moins un plan de traitement optimisé à durée d'irradiation escomptée inconnue et
b) on détermine la durée d'irradiation escomptée à partir d'au moins un plan de traitement et à l'aide de grandeurs caractéristiques d'accélérateur de particules, **caractérisé en ce que** les grandeurs caractéristiques d'accélérateur de particules comprennent au moins le temps d'accélération, la longueur de rétrodiffusion et des degrés d'intensité de l'émission des particules.

10. Produit de programme d'ordinateur suivant la revendication précédente, **caractérisé en ce que** les grandeurs de plan de traitement à partir desquelles on établit (2, 3, 4, 5) au moins un plan de traitement sont optimisées avant l'exploitation de la durée de traitement escomptée.

11. Produit de programme d'ordinateur suivant l'une des revendications précédentes 9 et 10,
**caractérisé en ce que**, comme autre stade, on effectue une sortie de la valeur de la durée de traitement escomptée à partir d'un plan de traitement exploité auparavant.

12. Produit de programme d'ordinateur suivant la revendication précédente,
**caractérisé en ce que** l'on propose et/ou sélectionne (7a) le plan de traitement exploité.
